# EUROPEAN PATENT APPLICATION

(11) **EP 3 597 296 A1**
(43) Date of publication of application: **22.01.2020**
(21) Application number: 18183978.8
(22) Date of filing: 17.07.2018
(51) Int. Cl.: B01L 3/00, C12M 3/06, C12M 1/42, B01F 13/00

(54) **MICROFLUIDIC DEVICE FOR APPLYING PRESSURE TO A CELL ASSEMBLY**

(71) Applicant: Technische Universität Wien, 1040 Wien (AT)
(72) Inventor: Ertl, Peter, 1190 Wien (AT); Rothbauer, Mario, 2511 Pfaffstätten (AT)
(74) Representative: Schwarz & Partner Patentanwälte OG

(57) **Abstract**

Microfluidic device (1) for applying pressure to a cell assembly (16), comprising a cell chamber (2), wherein the cell chamber (2) comprises:
an inlet (5) for introducing a cell assembly into the cell chamber (2),
a mechanical pressing means (13) for applying pressure to the cell assembly and being operable between a releasing position and a pressing position via an actuation line (7). The pressing means (13) has a cell assembly contact surface (4) facing the interior of the cell chamber (2), wherein the cell assembly contact surface (4) has at least one structured portion (14) and/or at least one sensor (18) and/or wherein the pressing means (13) comprises a actuation chamber (17), wherein an extension of the actuation chamber (17) is larger than an extension of the cell chamber (2).

## Description

The present invention relates to a microfluidic device for applying pressure to a cell assembly and methods for using said device.

Cell motility and cell migration, both important parameters for regenerative biological processes in vivo, is frequently tested using so-called wound healing assays. In addition, such assays are applied in cancer research to test the aggressiveness cancer cells to form metastasis and allow to test for drugs that inhibit metastasis and/or cancer growth. Also, these assays provide an effective method to assess the speed of tissue regeneration as well as quality of wound healing for tissue wounds (e.g. burn wounds), and may provide prognostic information about wound healing outcomes based on patient-derived sample material.

In general, cell migration involves the movement of cells in response to biological signals and environmental cues and plays a vital role in a variety of key physiological processes including immune cells recruitment, wound healing, and tissue repair as well as embryonic morphogenesis. For instance, in case of infections immune cells rapidly move from the lymph nodes via the circulation towards the infected site, while during wound healing cells steadily migrate to the injured tissue in a continuous attempt to repair the damage. In turn, abnormal cell migration resulting in the relocation of improper cell types to unsuitable tissue sites can lead to serious consequences such as mental retardation, cardiovascular disease, and arthritis as well as cancer metastasis and tumor formation. It has been recently recognized that only a better understanding of the complex, dynamic processes that govern cell migration will foster the development of novel therapeutic strategies to combat these diseases. A variety of approaches exist to induce wounds within a confluent cell monolayer, which can be separated into chemical (enzymatic), thermal (heating), optical (laser ablation) and mechanical removal of cells. An alternative method of creating defined depletion zones is based on the integration of electrodes to apply strong localized electrical fields to either lyse cells or prevent cell ingrowth (e.g. electric fence). Among these, the by far most popular cell depletion method is based on mechanical damage of cell surface layers, also called 'scratch assay', where a cell-free area within a cell monolayer is created by manually scratching the cell culture surface using either plastic pipette tips or specialized blades to forcefully remove adherent cells from a surface.

Nowadays a clear technological shift from two-dimensional cell based in vitro assays to physiologically more relevant three-dimensional in vitro models is occurring. Tissue engineering has fostered the development of a variety of three-dimensional culture techniques including hydrogel-based approaches (e.g. cell mono- and co-cultures embedded in synthetic and/or natural hydrogels) as well as cellular self-assembly (e.g. spheroid formation in hanging drops or round bottom microtiter plates). In addition, microfluidic devices that exploit actuation of cell cultures on-chip based on the integration of flexible membranes in microfluidic cell culture devices have improved differentiation capacities and physiological phenotypes by inducing controlled stretching or compression profiles.

Despite the above mentioned recent achievements of microfluidic two-dimensional cell culture systems, they still do not address the fact that in vivo cells coexist in three-dimensional communities that are influenced by spatial orientation of cells in a cell assembly and cell-to-cell contact within the extracellular matrix. It has been repeatedly demonstrated that the presence of a three-dimensional matrix promotes many biologically relevant functions not observed in two-dimensional monolayer cell cultures. Consequently, a transition from two-dimensional to three-dimensional cell cultures on-chip has gained momentum as an increasing number of reports have confirmed significant differences in the morphology, protein expression, differentiation, migration, functionality and viability of cells between three-dimensional and two-dimensional cell cultures.

A microfluidic device for two-dimensional cell cultures is known from EP 3 231 860 A1, for example. The microfluidic device comprises a cell chamber, which has an inlet for introducing the cell culture into the cell chamber and a mechanical pressing means for applying pressure to the cell culture to induce traumatic injuries to the cell culture to form cell free areas. The mechanical pressing means of this device is an inflatable membrane and is operable between a releasing position and a pressing position via an actuation line, wherein in the pressing position pressure / mechanical compression force is applied onto the cell culture via a cell culture contact surface.

Microfluidic devices known in the art (see e.g. EP 3 231 860 A1) allow to apply pressure only flatly to the cell culture inside the cell chamber resulting in the destruction and/or removal of cells due to the structure of the microfluidic device. Therefore, the cell culture can only be insured to a limited extend for testing traumatic events.
It is an object of the present invention to provide a method and a device to overcome the drawback of the state of the art and in particular provide a microfluidic device, which is capable of providing probes of cell assemblies with different types of injuries.

This object is solved by a microfluidic device for applying pressure to a cell assembly, comprising at least one cell chamber, wherein the at least one cell chamber comprises:
- an inlet for introducing a cell assembly into the cell chamber, and
- at least one mechanical pressing means for applying pressure to the cell assembly and being operable between a releasing position and a pressing position via an actuation line. The at least one pressing means has a cell assembly contact surface facing the interior of the cell chamber, wherein the cell assembly contact surface has at least one structured portion, preferably a micro-structured portion.

With the structured portion, preferably the micro-structured portion, applied to the cell assembly contact surface facing the interior of the cell chamber it is possible to generate impacts and injuries of different size, depth and shape in the cell assembly, wherein the size, depth and shape of the injuries/ impacts in the cell assembly depend on the shape and size of the structured portion and the pressure applied to the cell assembly. This has the advantage, that different types of injuries can be produced in the cell assembly, which enhances the producible variety of probes of injured cell assemblies. Furthermore, by using cornered structured portions pressure can be applied to the cell assembly in a more precise way, since the corners of the structured portion prevent the cell assembly from dodging away when the pressure is applied to the cell assembly. Accordingly, the reproducibility of the probes is enhanced, which is important for producing identical probes of cell assemblies and generating comparable results in using the probes.

In a preferred embodiment of the invention, the at least one pressing means has at least one sensor for sensing a property or state of the cell assembly within the cell chamber. This has the advantage, that the cell chamber can be monitored during the pressure application, whereby it is possible to react immediately to changes in the cell assembly during the pressure application and thus the cell assembly can be injured more precisely.

The at least one sensor provided on or within the at least one pressing means can be an electrochemical or optical biosensor. These sensors can be used to monitor or check the cell viability, cell metabolism, cell integrity, cell morphology, proliferation, cell-cell interaction, cell-matrix interaction and cells secretion and signaling.

Electrochemical (bio)sensors to be used according to the present invention include trans-endothelium electrical resistance (TEER) sensors, giant magnetoresistance (GMR) sensors, patch clamp sensors, amperometric and impedance sensors and cyclic voltammetry sensors. These sensors can be used to measure cell integrity, metabolism, uptake, membrane potential, etc. in a direct or indirect way. Electrochemical (bio) sensors are known from Wartmann D et al. (Front. Mater. 2 (2015): 60), for example.

Optical (bio)sensors to be used according to the present invention are able to detect luminsecence/fluorescence in order to determine the pH and/or the concentration of oxygen, glucose and lactate within the cell chamber of the device of the present invention. All these sensors and their settings are well known in the art.

Other optical (bio)sensor used in the present invention can be based on light scattering. In this context, the material of the at least one cell chamber is chosen to be preferably transparent and the microfluidic device is preferably arranged in a light scattering measuring housing in order to sense the property of state of the cell assembly within the cell chamber.

Another problem of microfluidic devices known in the art is that it is not possible to monitor the cell culture assembly during the application of the mechanical compression force.

Hence, it is a another object of the present invention to provide a method and a device to overcome the drawback of the state of the art and in particular provide a microfluidic devices which is capable of providing better and more precise probes of injured cell assemblies.

Therefore, the present invention further relates to a microfluidic device for applying pressure to a cell assembly, comprising at least one cell chamber, wherein the at least one cell chamber comprises:
- an inlet for introducing a cell assembly into the cell chamber,
- at least one mechanical pressing means for applying pressure to the cell assembly and being operable between a releasing position and a pressing position via an actuation line, wherein the at least one pressing means has a cell assembly contact surface facing the interior of the cell chamber and at least one sensor for sensing a property or state of the cell assembly within the cell chamber.

With the at least one sensor used in the present invention it is possible to measure different parameters of the cell assembly during the application of pressure to the cell assembly. With the measured parameters, the cell assembly can be monitored during the application of pressure to the cell assembly, whereby it is possible to react immediately to unwanted changes in the cell assembly during the pressure application and thus the cell assembly can be injured more precisely.

In a preferred embodiment of this aspect of the invention, the cell assembly contact surface has at least one structured portion, preferably a micro-structured portion. With the structured portion applied to the cell assembly contact surface facing the interior of the cell chamber it is possible to generate impacts in the cell assembly of different size, depth and shape depending on the size and shape of the structured portion and the pressure applied on the cell assembly. This has the advantage, that different types of injuries can be produced in the tissue, which enhances the producible variety of probes of cell assemblies. Furthermore, by using cornered structured portions pressure can be applied to a three-dimensional cell assembly in a more precise way, since the corners of the structured portion prevent the cell assembly from dodging away when the pressure is applied to the cell assembly. Accordingly, the reproducibility of the probes is enhanced, which is important for producing identical probes of cell assemblies and generating comparable results in using the probes.

In a preferred embodiment of the present invention the structured portion of the cell assembly contact surface is formed from at least one protrusion, preferably from at least two protrusions, more preferred from a plurality of protrusions, protruding into the interior of cell chamber and/or wherein the structured portion of the cell assembly contact surface is formed from at least one recess, preferably from at least two recesses, more preferred from a plurality of recesses. With a structured portion formed from at least two protrusions and/or at least two recesses it is possible to generate more impacts and thus injuries in one single cell assembly. If the micro fluidic device according to the present invention has at least two protrusions and/or recesses, the protrusions and/or recesses can either have the same shape and/or size or they can have a different shape and/or size.

In another preferred embodiment of the present invention the cross-section of the at least one protrusion, which cross-section is parallel to the cell assembly contact surface or transverse to the pressing direction of pressing means, decreases in direction towards the interior of the cell chamber. A protrusion having such a shape is favorably used for generating deep impacts/ injuries in the cell assembly. The at least one protrusion can be for example a cone, a truncated cone, a half sphere, a half ellipsoid, a pyramid or a truncated pyramid, etc., of different size and extension. In another preferred embodiment of the invention, the at least one protrusion can also be a cube, a cuboid and/or a strip.

In a preferred embodiment of the present invention the cross-section of the at least one protrusion at its distal end amounts to 2 cm², preferably to 1 cm², more preferably to 0.8 cm², more preferably to 0.5 cm², more preferably to 0.2 cm², more preferably to 0.1 cm², more preferably to 5 mm², more preferably even down to 1 mm² at the most, and/or wherein the cross-section of the at least one protrusion (15a-15i) at its proximal end amounts to at least 25 µm², preferably at least 50 µm², more preferably at least 100 µm².

In a further preferred embodiment of the present invention the at least one protrusion ends in a tip, preferably a sharp tip, and/or wherein the at least one protrusion is cone-shaped or pyramid-shaped. This has the advantage, that the pressure can be applied to the cell assembly punctured, wherein high-pressure forces can be applied to the cell assembly.

In another preferred embodiment of the present invention, the cell chamber is formed by a first wall and a second wall that is opposite to the first wall, wherein the cell assembly contact surface of pressing means forms at least a portion of the first wall. Preferably, the first wall and the second wall a parallel to each other. In another embodiment, the first and the second wall are inclined to each other.

In a preferred embodiment of the present invention the extension of the at least one protrusion in the direction perpendicular to the cell assembly contact surface amounts to at least 1%, preferably at least 10%, more preferred at least 30%, of the maximal distance between the first wall and the second wall and/or amounts to at least 5 µm, preferably at least 50 µm, more preferred at least 150 µm. Preferably, the extension of the at least one protrusion is chosen as a function of a kind of cells used in the cell assembly and/or a size and/or a shape of the cell assembly, on which pressure is applied.

In another preferred embodiment of the present invention at least a portion of the actuation line is formed by an actuation chamber which is separated from the cell chamber by a flexible membrane, wherein the proximal cross section of the at least one protrusion parallel to the membrane amounts to not more than 50%, preferably not more than 30% smaller, more preferred not more than 10%, of the overlapping area of actuation chamber and cell chamber. Preferably, the proximal cross section of the at least one protrusion is chosen as a function of a kind of cells used in the cell assembly and/or a size and/or a shape of the cell assembly, on which pressure is applied.

In a further preferred embodiment of the present invention, the at least one sensor is formed on or within the cell assembly contact surface. The at least one sensor can be a biochemical, chemical or electrical, electromagnetic, acoustic or electro-chemical or optical sensor. In the latter case connection cables of the at least one sensor are preferably guided through the membrane out of the microfluidic device. Preferably, the membrane comprises a guiding hole for the connection cables.

In a preferred embodiment of the present invention, the at least one sensor is a chemical and/or biological or conductivity sensors using optical, electrochemical, magnetic transducers. If the microfluidic device comprises a protrusion with a tip, the at least one sensor is preferably positioned as close as possible to the tip inside the flexible membrane or the protrusion.

In another preferred embodiment of the present invention, the at least one sensor converts a property or state of the cell assembly into a colour change and/or into a light transmission change and/or into a change of the absorption and/or emission spectrum of the sensor material and/or change in current/voltage/impedance/resistance. Preferably, the at least one sensor is capable of measuring temperature, electrical resistance, pressure, and/or stain etc. of the cell assembly during application of pressure to the cell assembly.

In a preferred embodiment of the present invention the at least one sensor is formed by a coating, wherein preferably the coating comprises a metal film, nanoparticles, microparticles, chemical indicators/dyes ("sensing compounds") and/or metal and/or metal oxides. The metal film can be a metal thin film, a multi-metal thin film or a metal alloy thin film, wherein a "thin film" may have a thickness of 5 to 2,000 nm, preferably 5 to 1,500 nm, more preferably 5 to 1,000 nm, more preferably 10 to 900 nm. These metal and metal alloy films can function as electrodes and may comprise or consist of chrome, gold, titan, silver, silver chloride, platinum or combinations thereof such as chrome and gold, titan and gold, silver and silver chloride. The use of thin films as defined above has the advantage, that the sensor can be configured very thin and small, wherein the presence of the at least on sensor in the microfluidic device does not affect the application of the pressure on the cell assembly.

It is particularly preferred to provide on the surface of the at least one pressing means microparticles and/or nanoparticles which may comprise or consist of at least one sensing compound. Said at least one sensing compound may react with at least one substance secreted by the cell assembly. For instance, sensing compounds may include porphyrins which are able to react with oxygen (see e.g. Quaranta M et al. (Bioanal Rev. 4(2012):115-157)). Other preferred sensing compounds may be pH sensitive. Preferred pH sensitive compounds may be fluorescein and its derivatives, seminaphthorhodafluor, 8-hydroxypyrene-1,3,6-trisulfonic acid sodium salt, hydroxycoumarins, BODIPY and azaBODIPY (BF₂-chelated tetraarylazadipyrromethene) dyes, cyanine-based probes, diketo-pyrrolo-pyrrole pigments, phenoxiazines and aminoperylene compounds.

In a preferred embodiment of the present invention, at least a portion of the actuation line is formed by an actuation chamber, which is separated from the cell chamber by the flexible membrane. Preferably, the actuation chamber is built by an inflatable membrane or by a hydraulic, magnetic, or pneumatic actuator.

Another problem of microfluidic devices known in the art is that with a three-dimensional cell assembly and particularly with a small three-dimensional cell assembly, there is always the risk that the cell assembly dodges to a side of the cell chamber when the mechanical pressing means are actuated from the releasing position into the pressing position. Therefore, either only small mechanical pressure forces are applied onto the cell assembly or in the worst case, no pressure is applied to the cell assembly.

It is a further object of the present invention to provide a method and a device to overcome the problems of the state of the art and in particular, to provide a microfluidic device, which is capable of providing better and more precise probes of injured cell assemblies.

Thus, another aspect of the present invention relates to a microfluidic device for applying pressure to a cell assembly comprising at least one cell chamber, wherein the at least one cell chamber comprises:
- an inlet for introducing cells into the cell chamber,
- at least one mechanical pressing means for applying pressure to the cell assembly and being operable between a releasing position and a pressing position via an actuation line, wherein the at least one pressing means is formed by a flexible membrane having a cell assembly contact surface facing the interior of the cell chamber,
at least a portion of the actuation line is formed by an actuation chamber which is separated from the cell chamber by the flexible membrane,
the inner extension of the actuation chamber in at least one direction, preferably in each direction parallel to the membrane is at least as large, preferably larger, than the inner extension of the cell chamber in the same direction(s).

Due to this configuration of the microfluidic device, the at least one pressing means reaches in each corner of the cell chamber when the at least one pressing means is in the pressing position. Thereby, the cell assembly cannot dodge to a side of the cell chamber when the mechanical pressing means are actuated from the releasing position into the pressing position and the pressure is applied to the cell assembly.

In another preferred embodiment, the cell chamber completely overlaps with the actuation chamber.

In a preferred embodiment of the present invention the inner extension of the actuation chamber in at least one direction, preferably in each direction parallel to the membrane is at least 1,3 times larger, preferably at least 1,8 times larger, than the inner extension of the cell chamber in the same direction(s). With this configuration, it can be made completely sure, that the flexible membrane reaches in each corner of the cell chamber when the flexible membrane is in the pressing position.

In a further preferred embodiment of the present the invention, the cell assembly contact surface of the at least one pressing means is formed by a flexible membrane.

In a preferred embodiment of the present invention the flexible membrane has a thickness between 10 µm and 500 µm, preferably between 200 µm and 300 µm, and/or wherein the membrane (3) comprises Polydimethylsiloxan (PDMS) and/or teflon. A thickness of the membrane between 10 µm and 500 µm, preferably between 200 µm and 300 µm, has the advantage that the membrane is flexible and still has enough resistance to transfer the pressure force to the cell assembly.

In a preferred embodiment of the present the invention the inner surface of the cell chamber wall that is opposite to the cell assembly contact surface of pressing means is substantially planar. In another preferred embodiment of the first aspect, second aspect and/or third aspect of the invention, the inner surface of the cell chamber wall that is opposite to the cell assembly contact surface is concave, convex or has the form of a half shell.

In a preferred embodiment of the present invention the inner surface of the cell chamber wall that is opposite to the cell assembly contact surface of the pressing means is coated with at least one polypeptide and/or peptide, preferably with at least one cell adhesion promoter, which is preferably selected from the group consisting of fibronectin, fibrinogen, gelatin, collagen, laminin, poly-D-lysine and mixtures thereof.

In another preferred embodiment of the present invention the operation range of the cell assembly contact surface of pressing means has essentially circular or polygonal shape and/or wherein the operation range of the cell contact surface of pressing means has an area between 0.2 mm² and 5 mm², preferably 0.5 mm² and 3 mm². Preferably, the operation range of the cell contact surface of the pressing means is chosen as a function of a kind of cells in the cell assembly and/or size of the cell assembly.

In a preferred embodiment of the present invention the microfluidic device has, at least in the area of the at least one cell chamber, a sandwich structure with a top layer, an intermediate layer and a bottom layer, wherein the mechanical pressing means is formed by the intermediate layer. This allows a reliable, space-saving and cost-effective construction.

In a further preferred embodiment of the present invention, the sidewalls of the cell chamber are formed by the bottom layer and/or by the intermediate layer. The number of pieces that have to be assembled during production of the device of the present invention may be significantly reduced.

In a preferred embodiment of the present invention, at least a portion of the actuation line is formed within the top layer. The integration of the actuation functionality into the top layer further reduces constructional efforts.

In a preferred embodiment of the present invention, the actuation line that acts on the mechanical pressing means may be a pressure and/or vacuum transmitting line, such as a pneumatic line or a fluid line (using a medium such as gas or oil) but may also be an electric actuation line for driving the mechanical pressing means via an electric, magnetic and/or electromagnetic field. In fact, any type of actuation capable of moving the mechanical pressing means from the releasing position to the pressing position and/or vice versa would be possible for the purpose of the present invention. This means that also an externally applied magnetic and/or electromagnetic field capable to move the pressing means can be used. In such a case the pressing means preferably comprise ferromagnetic particles or are coated at least partially, preferably completely, with a ferromagnetic coating. The magnetic and/or electromagnetic field can be provided using one or more magnets and/or one or more electromagnets which are positioned nearby the mechanical pressing means. The use of electromagnets is particularly preferred because it allows to control the magnetic field by applying electricity so that the position of these electromagnets must not be changed after moving the pressing means.

In a further preferred embodiment of the present invention, the at least one cell chamber comprises an outlet, wherein a flow path is defined in the cell chamber between the inlet and the outlet. This has the advantage, that the cell chamber can be flushed with a fluid, e.g. gas or a liquid.

In another preferred embodiment of the present invention, in a cross section perpendicular to the flow path lateral areas of the cell chamber are outside of the operation range of the pressing means. This allows maintaining a flow rate also in the pressing position of the mechanical pressing means.

In a preferred embodiment of the present invention, the microfluidic device has a flat cross section and/or elongated shape. This has the advantage that a plurality of microfluidic devices can be arranged space saving parallel to each other. Please note, that the shape of the microfluidic device may be any one. However, it is particularly preferred that the cell chamber has an elongated shape.

In another preferred embodiment of the present invention, the actuation chamber as a circular shape.

In a preferred embodiment of the present invention the height of the cell chamber is smaller than 1 mm and/or wherein the width of the cell chamber amounts between 200 µm and 5 mm and/or wherein the length of the cell chamber amounts between 2 mm and 10 cm.

In a preferred embodiment of the present invention the microfluidic device comprises a plurality of cell chambers, preferably at least two, more preferably at least three, more preferably at least six, more preferably at least 12, more preferably at least 24, more preferably at least 48, more preferably at least 96, cell chambers.

Another aspect of the present invention relates to a method of applying pressure to a cell assembly within a microfluidic device according to the embodiments listed above comprising the steps of:
- (a) placing the at least one cell assembly in the cell chamber,
- (b) bringing pressing means via actuation line in a pressing state, in which the cell assembly contact surface exerts a pressure on the cell assembly, and
- (c) bringing pressing means in a releasing state.

In a first step (a) a cell assembly is placed into the at least one cell chamber via the inlet. Preferably, the cell assembly is supplied to the at least on cell chamber via a fluid. In a second step (b) the cell assembly is contacted by pressing means in order to expose the cell assembly to pressure which may simulate an injury or trauma. This is achieved by bringing the mechanical pressing means in a pressing state via the actuation line, whereby the cell assembly contact surface exerts a pressure on the cell assembly. In a last step (c) the mechanical pressing means are brought back in the releasing state.

According to another preferred embodiment of the present invention during step (b) the cell assembly contact surface of the pressing means is moved into the interior of the cell chamber. Preferably, the cell assembly contact surface is moved into the interior of the cell chamber in such a way, that it reaches each area of the cell chamber to insure that pressure is applied to the cell assembly.

According to another preferred embodiment of the present invention the cell chamber is formed by a first wall and a second wall that is opposite to the first wall, wherein the cell assembly contact surface of pressing means forms at least a portion of the first wall, wherein during all steps (a) to (c) the cell assembly contact surface of pressing means is kept at a distance from the second wall. This has the advantage, that the cell assembly is not divided into two pieces, when the pressure is applied to the cell assembly. Preferably the distance, at which the cell contact surface is kept from the second wall amounts to at least 100µm, preferably at least 250 µm, preferably at least 500 µm, more preferred to at least 1 mm, and/or wherein the minimal distance, at which the cell contact surface is kept from the second wall amounts to at least 0.1%, preferably at least 1%, more preferred to at least 10%, more preferred to at least 50% of the maximal distance between the first wall and the second wall.

According to another preferred embodiment of the present invention, the pressure exerted on the cell assembly by pressing means is adjusted by pneumatic, hydraulic or electromechanical means. The pneumatic or hydraulic means can be a pneumatic or hydraulic pressure controller which is adjustable by hand or which is hooked up to a microcontroller. The electromechanical means if preferably a microcontroller or an electrical element controlled by hand or via a microcontroller, like a tuneable resister, etc.

According to another preferred embodiment of the present invention, the cell assembly comprises eukaryotic cells, preferably mammalian or human cells.

According to another preferred embodiment of the present invention, the cell assembly is selected from the group of a tissue, an organ, an organoid, a spheroid, a 3D-hydrogel based cell culture, a 3D-based hydrogel co-culture system, and multicellular assemblies

These and further advantageous embodiments of the invention will be explained based on the following description and the accompanying drawings. The person skilled in the art will understand that various embodiments may be combined.
Figures 1 to 3 show in a cross-sectional view an embodiment of a microfluidic device according to the invention in different operational stages.
Figure 4 shows in top view an assembly having four microfluidic devices according to figures 1 to 3.
Figures 5 to 13 show in cross-sectional section views various embodiments of a microfluidic device according to the invention.
Figure 14 shows in a cross-sectional view an embodiment of a microfluidic device according to the invention.

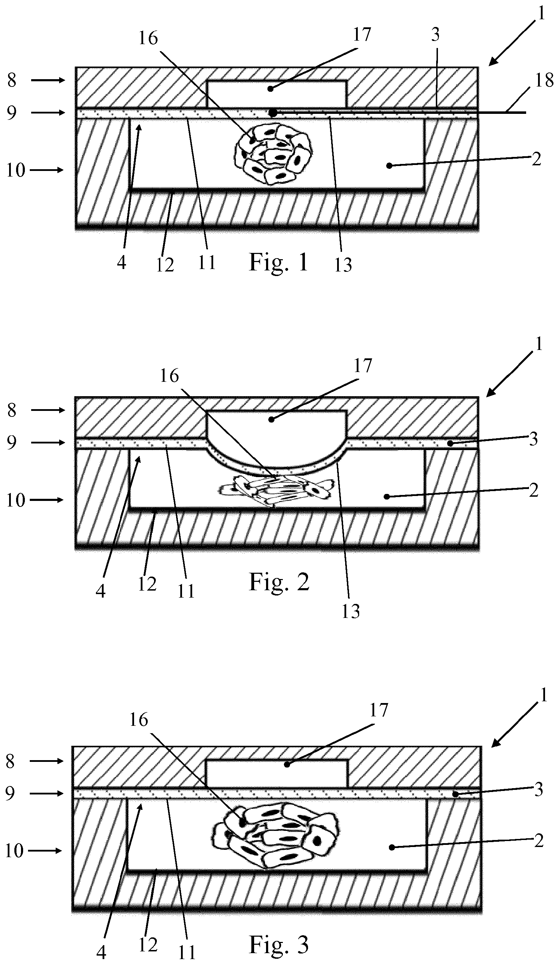

Figures 1 to 3 show in a cross-sectional view an embodiment of a microfluidic device 1 according to the invention in different operational stages. The microfluidic device 1 of figures 1 to 3 comprises a bottom layer 10 having a cell chamber 2, an intermediate layer 9 formed by a flexible membrane 3 and a top layer 8 having a actuation chamber 17. The flexible membrane 3 is in a sealing connection with the bottom layer 10 and the top layer 8.

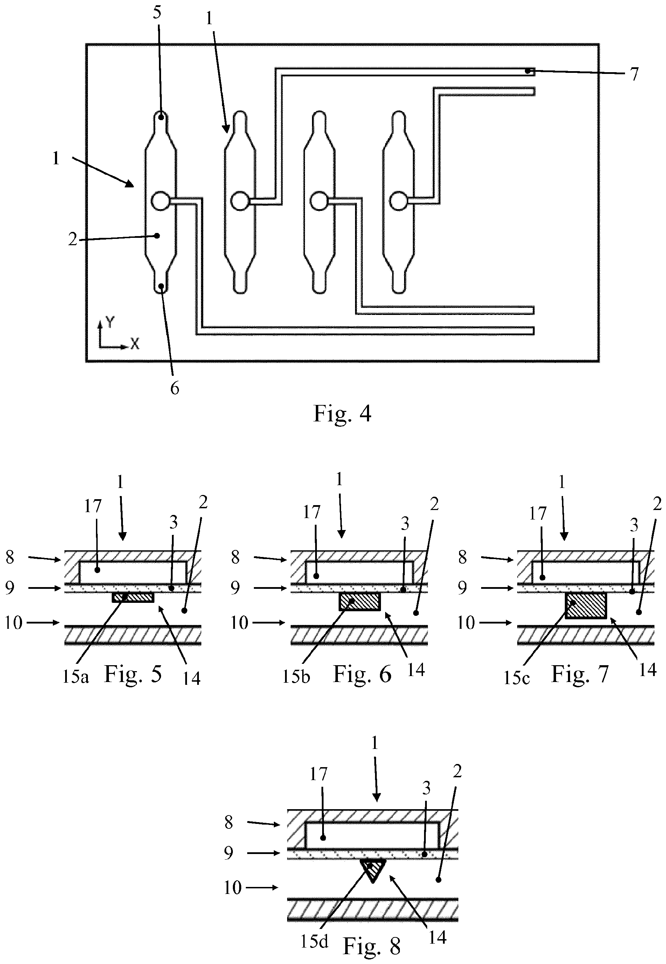

The bottom layer 10 comprises an inlet 5 for introducing fluid and/or a cell assembly into the cell chamber 2 and an outlet 6. Please see figure 4. The flexible membrane 3 has a cell contact surface 4, which faces the interior of the cell chamber 2.

The actuation chamber 17 is connected to an actuation line 7. Via the actuation line 7, a fluid like air, nitrogen, carbon dioxide, water, oil, etc. can be forwarded into the actuation chamber 17. Please see figure 4.

The bottom layer 10 comprises a second wall 12 and the flexible membrane 3 forms in the area of the cell chamber 2 a first wall 11, which is opposite to the second wall 12.

The flexible membrane 3 forms a pressing means 13. By applying pressured fluid via the actuation line 7 to the actuation chamber 17, the actuation chamber 17 is inflated and the pressing means 13 is operated from a releasing position into a pressing position.

The inner surface of the second wall 12 is substantially planar. Alternatively, the inner surface of the second wall 12 can also be concave or convex.

The inner surface of the second wall 12 may be coated with at least one polypeptide and/or peptide, preferably with at least one cell adhesion promoter, which is preferably selected from the group consisting of fibronectin, fibrinogen, gelatin, collagen, laminin, poly-D-lysine and mixtures thereof.

Moreover, the microfluidic device 1 of figures 1 to 3 comprises a sensor 18, which is provided inside the flexible membrane 3. In this embodiment, the sensor 18 is a sensor for measuring the electrical resistance of the cell assembly. For the sake of simplicity, the sensor 18 is only shown in figure 1. The connection cables of the sensor 18 are guided inside the flexible membrane 3 in a guiding hole, which is not shown in the figures.

In another embodiment, the sensor 18 can also be provided in or on the wall 12.

With reference to figures 1 to 3, a pressure application to a cell assembly 16 located in the cell chamber 2 to injure the cell assembly 16 will now be described in detail.

In a first step, the cell assembly 16 is introduced into the cell chamber 2 via the inlet 5. Preferably, the cell assembly 16 is transported into the cell chamber 2 via a fluid, preferably a liquid containing nutrients. After the introduction of the cell assembly 16 into the cell chamber 2, the pressing means 13 is forced from the releasing position into a pressing position by pressure transmitted via the actuation line 7 to the actuation chamber 17. The pressure transmitted via the actuation line 7 to the actuation chamber 17 is preferably controlled by a pressure regulator. By forcing the pressing means 13 from the releasing position into the pressing position, the flexible membrane 3 is stretched and thus pre-stressed. Please see figure 2. During this procedure, the electrical resistance of the cell assembly 16 is currently monitored with the sensor 18, whereby it is possible to stop the application of the pressure if the electrical resistance of the cell assembly 16 exceeds a predetermined critical threshold value.

After a predefined time, the pressure is released in the cell chamber 2, whereby the pressing means moves back into the releasing position due to the pre-stressed flexible membrane 3. In another embodiment, the movement of the pressing means 13 from the pressing position to the releasing position is enhanced by applying a vacuum to the actuation line 7, thereby sucking the membrane away from the cell assembly 16. In a last step fluid is supplied via the inlet 5 to the cell chamber 2, whereby the injured cell assembly 16 is discharged from the cell chamber 2 through the outlet. After separating the cell assembly 16 from the fluid, the cell assembly 16 can be provided to a further process.

Figure 4 shows in top view an assembly having four microfluidic devices 1 according to figures 1 to 3.

By supplying fluid to the cell chamber 2 via the inlet 5 a flow path establishes between the inlet 5 and the outlet 6 through the cell chamber 2. In a cross section perpendicular to the flow path lateral areas of the cell chamber 2 are outside of the operation range of the mechanical pressing means 13.

Figures 5 to 13 show in cross-sectional views various embodiments of a microfluidic device 1 according to the invention.

In contrast to the micro fluidic device of figures 1 to 3 the micro fluidic devices of figures 5 to 13 do not comprise a sensor 18, but comprise a structured portion 14. In another embodiment of the micro fluidic device 1 the micro fluidic device 1 comprises a sensor 18 as well as a structured portion 14.

Figures 5 to 7 respectively show a micro fluidic device 1 having a structured portion 14 formed by a protrusion in the form of a cuboid 15a, 15b or 15c. Each cuboid 15a, 15b, 15c is connected with the flexible membrane 3. The cuboids 15a, 15b and 15c differ in their height from each other. Preferably, the height of the cuboids 15a, 15b and 15c is chosen according to a kind of cells used in the cell assembly 16 and/or a size and/or shape of the cell assembly 16.

Figure 8 shows a micro fluidic device 1 having a structured portion 14 formed by a protrusion in the form of a pyramid 15d. The pyramid 15d is connect with the flexible membrane 3. Due to the form of the pyramid 15d, a cross section of the pyramid 15d, which is parallel to the cell assembly contact surface 4, decreases in the direction towards the interior of the cell chamber 2. The pyramid 15d is used to make deep impacts/ injuries into the cell assembly 16.

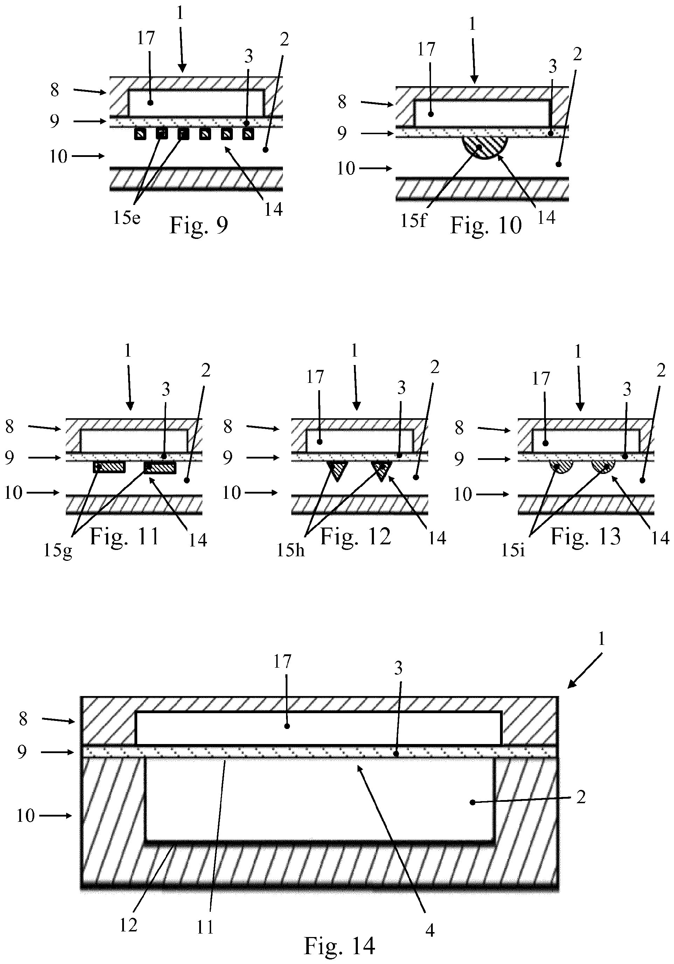

Figure 9 shows a micro fluidic device 1 having a structured portion 14 formed by a plurality of protrusions in the form of cubes 15e. The cubes 15a are connected to the flexible membrane 3 spaced apart from each other.

Figure 10 shows a micro fluidic device 1 having a structured portion 14 formed by a protrusion in the form of a hemisphere 15f. The hemisphere 15f is connected to the flexible membrane 3.

Figure 11 shows a micro fluidic device 1 having a structured portion 14 formed by two protrusions in the form of two cuboids 15g. The cuboids 15g are connected with the flexible membrane 3 spaced apart from each other.

Figure 12 shows a micro fluidic device 1 having a structured portion 14 formed by two protrusions in the form of two cones 15h. The cones 15h are connected with the flexible membrane 3 spaced apart from each other.

Figure 13 shows a micro fluidic device 1 having a structured portion 14 formed by two protrusions in the form of two hemispheres 15i. The hemispheres 15i are connected with the flexible membrane 3 spaced apart from each other.

A micro fluidic device 1 having two protrusion has the advantage, that it is possible to produce two impacts/ injuries in the cell assembly 16 simultaneously.

Preferably, an adhesive is used to permanently connect the protrusions with the flexible membrane 3.

Figure 14 shows in a cross-sectional view an embodiment of a micro fluidic device 1 according to the invention.

In contrast to the micro fluidic device of figures 1 to 3 the micro fluidic device 1 of figure 14 does not comprise a sensor 18, but comprises an actuation chamber 17 with an inner extension lager than an inner extension of the cell chamber (2) in a direction perpendicular to an imaginary line between the inlet 5 and the outlet 6 and parallel to the flexible membrane 3. The actuation chamber 17 is circular. Please note, that the device 1 of figure 14 can also include at least one structured portion 14, preferably a micro-structured portion, and/or a sensor 18.

### Embodiments:

1. Microfluidic device (1) for applying pressure to a cell assembly (16), comprising at least one cell chamber (2), wherein the at least one cell chamber (2) comprises:
   - an inlet (5) for introducing a cell assembly (16) into the cell chamber (2),
   - at least one mechanical pressing means (13) for applying pressure to the cell assembly (16) and being operable between a releasing position and a pressing position via an actuation line (7),
   wherein the at least one pressing means (13) has a cell assembly contact surface (4) facing the interior of the cell chamber (2), wherein the cell assembly contact surface (4) has at least one structured portion (14), preferably a micro-structured portion.
2. Microfluidic device (1) according to embodiment 1, wherein the at least one pressing means (13) has at least one sensor (18) for sensing a property or state of the cell assembly (16) within the cell chamber (2).
3. Microfluidic device (1) for applying pressure to a cell assembly (16), comprising at least one cell chamber (2), wherein the at least one cell chamber (2) comprises:
   - an inlet (5) for introducing a cell assembly (16) into the cell chamber (2),
   - at least one mechanical pressing means (13) for applying pressure to the cell assembly and being operable between a releasing position and a pressing position via an actuation line (7),
   wherein the at least one pressing means (13) has a cell assembly contact surface (4) facing the interior of the cell chamber (2) and at least one sensor (18) for sensing a property or state of the cell assembly (16) within the cell chamber (2).
4. Microfluidic device (1) according to embodiment 3, wherein the cell assembly contact surface (4) has at least one structured portion (14), preferably a micro-structured portion.
5. Microfluidic device according to embodiment 1, 2 or 4, wherein the structured portion (14) of the cell assembly contact surface (4) is formed from at least one protrusion (15a-15i), preferably from at least two protrusions (15a-15i), more preferred from a plurality of protrusions (15a-15i), protruding into the interior of cell chamber (2) and/or wherein the structured portion (14) of the cell assembly contact surface (4) is formed from at least one recess, preferably from at least two recesses, more preferred from a plurality of recesses.
6. Microfluidic device according to embodiment 5, wherein the cross-section of the at least one protrusion (15d, 15f, 15h, 15i), which cross-section is parallel to the cell assembly contact surface (4) or transverse to the pressing direction of pressing means (13), decreases in direction towards the interior of the cell chamber (2).
7. Microfluidic device according to embodiment 5 or 6, wherein the cross-section of the at least one protrusion (15a-15i) at its distal end amounts to 2 cm², preferably to 1 cm², more preferably to 0.8 cm², more preferably to 0.5 cm², more preferably to 0.2 cm², at the most, and/or wherein the cross-section of the at least one protrusion (15a-15i) at its proximal end amounts to at least 25 µm², preferably at least 50 µm², more preferably at least 100 µm².
8. Microfluidic device according to any one of embodiments 5 to 7, wherein the at least one protrusion (15d, 15f, 15h, 15i) ends in a tip, preferably a sharp tip, and/or wherein the at least one protrusion (15d, 15h) is cone-shaped or pyramid-shaped.
9. Microfluidic device according to one of embodiments 1 to 8, wherein the cell chamber (2) is formed by a first wall (11) and a second wall (12) that is opposite to the first wall (11), wherein the cell assembly contact surface (4) of pressing means (13) forms at least a portion of the first wall (11).
10. Microfluidic device according to embodiment 9, wherein the extension of the at least one protrusion (15a-15i) in direction perpendicular to the cell assembly contact surface (4) amounts to at least 1%, preferably at least 10%, more preferred at least 30%, of the maximal distance between the first wall (11) and the second wall (12) and/or amounts to at least 5 µm, preferably at least 50 µm, more preferred at least 150 µm.
11. Microfluidic device according to any one of embodiments 5 to 10, wherein at least a portion of the actuation line (7) is formed by an actuation chamber (17) which is separated from the cell chamber (2) by a flexible membrane (3), wherein the proximal cross section of the at least one protrusion (15a-15i) parallel to the membrane (3) amounts to not more than 50%, preferably not more than 30% smaller, more preferred not more than 10%, of the overlapping area of actuation chamber (17) and cell chamber (2).
12. Microfluidic device according to any one of embodiments 2 to 11, wherein the at least one sensor (18) is formed on or within the cell assembly contact surface (4).
13. Microfluidic device according to any one of embodiments 2 to 12, wherein the at least one sensor (18) is a chemical and/or biological or conductivity sensors using optical, electrochemical, magnetic transducers.
14. Microfluidic device according to any one of embodiments 2 to 13, wherein the at least one sensor (18) converts a property or state of the cell assembly (16) into a color change and/or into a light transmission change and/or into a change of the absorption and/or emission spectrum of the sensor material and/or change in current/voltage/impedance/resistance.
15. Microfluidic device according to to any one of embodiments 2 to 14, wherein the at least one sensor (16) is formed by a coating, wherein preferably the coating comprises of nanoparticles/ microparticles, chemical indicators/dyes and/or metal and/or metal oxides.
16. Microfluidic device according to any one of embodiments 1 to 15, wherein at least a portion of the actuation line (7) is formed by an actuation chamber (17) which is separated from the cell chamber (2) by the flexible membrane (3).
17. Microfluidic device (1) for applying pressure to a cell assembly (16) comprising at least one cell chamber (2), wherein the at least one cell chamber (2) comprises:
   - an inlet (5) for introducing cells into the cell chamber (2),
   - at least one mechanical pressing means (13) for applying pressure to the cell assembly and being operable between a releasing position and a pressing position via an actuation line (7),
   wherein
   the at least one pressing means (13) is formed by a flexible membrane (3) having a cell assembly contact surface (4) facing the interior of the cell chamber (2), at least a portion of the actuation line (7) is formed by an actuation chamber (17) which is separated from the cell chamber (2) by the flexible membrane (3), the inner extension of the actuation chamber (17) in at least one direction, preferably in each direction parallel to the membrane (3) is at least as large, preferably larger, than the inner extension of the cell chamber (2) in the same direction(s).
18. Microfluidic device according to embodiment 17, wherein the cell chamber (2) completely overlaps with the actuation chamber (17).
19. Microfluidic device according to embodiment 17 or 18, wherein the inner extension of the actuation chamber (17) in at least one direction, preferably in each direction parallel to the membrane (3) is at least 1,3 times larger, preferably at least 1,8 times larger, than the inner extension of the cell chamber (2) in the same direction(s).
20. Microfluidic device according to any one of embodiments 1 to 19, wherein the cell assembly contact surface (4) of the at least one pressing means (13) is formed by a flexible membrane (3).
21. Microfluidic device according to embodiment 20, wherein the membrane (3) has a thickness between 10 µm and 500 µm, preferably between 200 µm and 300 µm, and/or wherein the membrane (3) comprises Polydimethylsiloxan (PDMS) and/or teflon.
22. Microfluidic device according to any one of embodiments 1 to 21, wherein the inner surface of the cell chamber wall (12) that is opposite to the cell assembly contact surface (4) of pressing means (13) is substantially planar.
23. Microfluidic device according to any one of embodiments 1 to 22, wherein the inner surface of the cell chamber wall (12) that is opposite to the cell assembly contact surface (4) of the pressing means (13) is coated with at least one polypeptide and/or peptide, preferably with at least one cell adhesion promoter, which is preferably selected from the group consisting of fibronectin, fibrinogen, gelatin, collagen, laminin, poly-D-lysine and mixtures thereof.
24. Microfluidic device according to any one of embodiments 1 to 23, wherein the operation range of the cell assembly contact surface (4) of pressing means (13) has essentially circular or polygonal shape and/or wherein the operation range of the cell contact surface (4) of pressing means (13) has an area between 0.2 mm² and 5 mm², preferably 0.5 mm² and 3 mm².
25. Microfluidic device according to any one of embodiments 1 to 24, wherein the microfluidic device (1) has, at least in the area of the at least one cell chamber (2), a sandwich structure with a top layer (8), an intermediate layer (9) and a bottom layer (10), wherein the mechanical pressing means (13) is formed by the intermediate layer (9).
26. Microfluidic device according to embodiment 25, wherein side walls of the cell chamber (2) are formed by the bottom layer (10) and/or by the intermediate layer (9).
27. Microfluidic device according to embodiment 25 or 26, wherein at least a portion of the actuation line (7) is formed within the top layer (8).
28. Microfluidic device according to any one of embodiments 1 to 27, wherein the actuation line (7) is a pneumatic line, a fluid line, an electric line, a magnetic line or an electromagnetic line.
29. Microfluidic device according to any one of embodiments 1 to 28, wherein the at least one cell chamber (2) comprises an outlet (6), wherein a flow path is defined in the cell chamber (2) between the inlet (5) and the outlet (6).
30. Microfluidic device according to embodiment 29, wherein in a cross section perpendicular to the flow path lateral areas of the cell chamber (2) are outside of the operation range of the pressing means (13).
31. Microfluidic device according to any one of embodiments 1 to 30, wherein the microfluidic device (1) has a flat cross section and/or elongated shape.
32. Microfluidic device according to any one of embodiments 1 to 31, wherein the height of the cell chamber (2) is smaller than 1 mm and/or wherein the width of the cell chamber (2) amounts between 200 µm and 5 mm and/or wherein the length of the cell chamber (2) amounts between 2 mm and 10 cm.
33. Microfluidic device according to any one of embodiments 1 to 32, wherein the microfluidic device (1) comprises a plurality of cell chambers (2), preferably at least two, more preferably at least three, more preferably at least six, more preferably at least 12, more preferably at least 24, more preferably at least 48, more preferably at least 96, cell chambers (2).
34. Method of applying pressure to a cell assembly (16) within a microfluidic device (1) according to any one of embodiments 1 to 33 comprising the steps of:
   - placing the at least one cell assembly in the cell chamber (2),
   - bringing pressing means (13) via actuation line (7) in a pressing state, in which the cell assembly contact surface (4) exerts a pressure on the cell assembly,
   - bringing pressing means (13) in a releasing state.
35. Method according to embodiment 34, wherein during step (b) the cell assembly contact surface of pressing means (13) is moved into the interior of the cell chamber (2).
36. Method according to embodiment 34 or 35, wherein the cell chamber (2) is formed by a first wall (11) and a second wall (12) that is opposite to the first wall (11), wherein the cell assembly contact surface of pressing means (13) forms at least a portion of the first wall (11), wherein during all steps (a) to (c) the cell assembly contact surface (4) of pressing means (13) is kept at a distance from the second wall (12).
37. Method according to embodiment 36,
   wherein the distance, at which the cell contact surface (4) is kept from the second wall (12) amounts to at least 100µm, preferably at least 250 µm, preferably at least 500 µm, more preferred to at least 1 mm,
   and/or wherein the minimal distance, at which the cell contact surface (4) is kept from the second wall (12) amounts to at least 0.1%, preferably at least 1%, more preferred to at least 10%, more preferred to at least 50% of the maximal distance between the first wall (11) and the second wall (12).
38. Method according to one of the embodiments 32 to 37, wherein the pressure exerted on the cell assembly (16) by pressing means (13) is adjusted by pneumatic, hydraulic or electromechanical means.
39. Method according to one of the embodiments 32 to 38, wherein the cell assembly comprises eukaryotic cells, preferably mammalian or human cells.
40. Method according to one of the embodiments 32 to 38, wherein the cell assembly is selected from the group of a tissue, an organ, an organoid, a spheroid, a 3D-hydrogel based cell culture, a 3D-based hydrogel co-culture system, and multicellular assemblies.

### List of reference signs

- 1: microfluidic device
- 2: cell chamber
- 3: flexible membrane
- 4: cell contact surface
- 5: inlet
- 6: outlet
- 7: actuation line
- 8: top layer
- 9: intermediate layer
- 10: bottom layer
- 11: first wall
- 12: second wall
- 13: pressing means
- 14: structured portion
- 15a-15i: protrusion
- 16: cell assembly
- 17: actuation chamber
- 18: sensor

## Claims

1. Microfluidic device (1) for applying pressure to a cell assembly (16), comprising at least one cell chamber (2), wherein the at least one cell chamber (2) comprises:
- an inlet (5) for introducing a cell assembly (16) into the cell chamber (2),
- at least one mechanical pressing means (13) for applying pressure to the cell assembly (16) and being operable between a releasing position and a pressing position via an actuation line (7),
wherein the at least one pressing means (13) has a cell assembly contact surface (4) facing the interior of the cell chamber (2), wherein the cell assembly contact surface (4) has at least one structured portion (14), preferably a micro-structured portion.

2. Microfluidic device (1) according to claim 1, wherein the at least one pressing means (13) has at least one sensor (18) for sensing a property or state of the cell assembly (16) within the cell chamber (2).

3. Microfluidic device (1) for applying pressure to a cell assembly (16), comprising at least one cell chamber (2), wherein the at least one cell chamber (2) comprises:
- an inlet (5) for introducing a cell assembly (16) into the cell chamber (2),
- at least one mechanical pressing means (13) for applying pressure to the cell assembly and being operable between a releasing position and a pressing position via an actuation line (7),
wherein the at least one pressing means (13) has a cell assembly contact surface (4) facing the interior of the cell chamber (2) and at least one sensor (18) for sensing a property or state of the cell assembly (16) within the cell chamber (2).

4. Microfluidic device (1) according to claim 3, wherein the cell assembly contact surface (4) has at least one structured portion (14), preferably a micro-structured portion.

5. Microfluidic device according to claim 1, 2 or 4, wherein the structured portion (14) of the cell assembly contact surface (4) is formed from at least one protrusion (15a-15i), preferably from at least two protrusions (15a-15i), more preferred from a plurality of protrusions (15a-15i), protruding into the interior of cell chamber (2) and/or wherein the structured portion (14) of the cell assembly contact surface (4) is formed from at least one recess, preferably from at least two recesses, more preferred from a plurality of recesses.

6. Microfluidic device according to claim 5, wherein the cross-section of the at least one protrusion (15d, 15f, 15h, 15i), which cross-section is parallel to the cell assembly contact surface (4) or transverse to the pressing direction of pressing means (13), decreases in direction towards the interior of the cell chamber (2).

7. Microfluidic device according to any one of claims 5 to 6, wherein at least a portion of the actuation line (7) is formed by an actuation chamber (17) which is separated from the cell chamber (2) by a flexible membrane (3), wherein the proximal cross section of the at least one protrusion (15a-15i) parallel to the membrane (3) amounts to not more than 50%, preferably not more than 30% smaller, more preferred not more than 10%, of the overlapping area of actuation chamber (17) and cell chamber (2).

8. Microfluidic device according to any one of claims 2 to 7, wherein the at least one sensor (18) is a chemical and/or biological or conductivity sensors using optical, electrochemical, magnetic transducers.

9. Microfluidic device (1) for applying pressure to a cell assembly (16) comprising at least one cell chamber (2), wherein the at least one cell chamber (2) comprises:
- an inlet (5) for introducing cells into the cell chamber (2),
- at least one mechanical pressing means (13) for applying pressure to the cell assembly and being operable between a releasing position and a pressing position via an actuation line (7),
wherein
the at least one pressing means (13) is formed by a flexible membrane (3) having a cell assembly contact surface (4) facing the interior of the cell chamber (2), at least a portion of the actuation line (7) is formed by an actuation chamber (17) which is separated from the cell chamber (2) by the flexible membrane (3), the inner extension of the actuation chamber (17) in at least one direction, preferably in each direction parallel to the membrane (3) is at least as large, preferably larger, than the inner extension of the cell chamber (2) in the same direction(s).

10. Microfluidic device according to claim 9, wherein the cell chamber (2) completely overlaps with the actuation chamber (17).

11. Microfluidic device according to any one of claims 1 to 10, wherein the cell assembly contact surface (4) of the at least one pressing means (13) is formed by a flexible membrane (3).

12. Microfluidic device according to any one of claims 1 to 11, wherein the inner surface of the cell chamber wall (12) that is opposite to the cell assembly contact surface (4) of the pressing means (13) is coated with at least one polypeptide and/or peptide, preferably with at least one cell adhesion promoter, which is preferably selected from the group consisting of fibronectin, fibrinogen, gelatin, collagen, laminin, poly-D-lysine and mixtures thereof.

13. Microfluidic device according to any one of claims 1 to 12, wherein the actuation line (7) is a pneumatic line, a fluid line, an electric line, a magnetic line or an electromagnetic line.

14. Method of applying pressure to a cell assembly (16) within a micro fluidic device (1) according to any one of claims 1 to 33 comprising the steps of:
- placing the at least one cell assembly in the cell chamber (2),
- bringing pressing means (13) via actuation line (7) in a pressing state, in which the cell assembly contact surface (4) exerts a pressure on the cell assembly,
- bringing pressing means (13) in a releasing state.

15. Method according to claim 14, wherein during step (b) the cell assembly contact surface of pressing means (13) is moved into the interior of the cell chamber (2).
